**Europäisches Patentamt**

**European Patent Office**

**Office européen des brevets**

(19)

(11) Numéro de publication: **0 253 740**
**B1**

(12) **FASCICULE DE BREVET EUROPEEN**

(45) Date de publication du fascicule du brevet:
30.05.90

(51) Int. Cl.⁵: **C07C 323/58**

(21) Numéro de dépôt: 87401670.2

(22) Date de dépôt: 16.07.87

(54) Procédé de préparation d'une solution aqueuse du sel de sodium de la méthionine.

(30) Priorité: 17.07.86 FR 8610399

(43) Date de publication de la demande:
20.01.88 Bulletin 88/3

(45) Mention de la délivrance du brevet:
30.05.90 Bulletin 90/22

(84) Etats contractants désignés:
AT BE CH DE ES FR GB IT LI LU NL SE

(56) Documents cités:
FR-A- 934 407
FR-A- 1 532 723
GB-A- 1 199 614
US-A- 2 642 459

(73) Titulaire: **RHONE-POULENC NUTRITION ANIMALE, Rue Marcel Lingot, F-03600 Commentry(FR)**

(72) Inventeur: **Giraud, Jean, Route de Commentry, F-03310 Néris-Les-Bains(FR)**

(74) Mandataire: **Le Pennec, Magali et al, RHONE-POULENC SANTE, Service Brevets, 20 Avenue Raymond Aron, F-92165 Antony Cédex(FR)**

## Description

La présente invention concerne un procédé de préparation d'une solution aqueuse du sel de sodium de la méthionine utilisable comme additif dans les aliments pour les animaux d'élevage.

La méthionine, qui est un aminoacide essentiel, est utilisée à des doses faibles, généralement inférieures à 1 % comme additif dans les aliments pour animaux. Il est particulièrement important de pouvoir réaliser une dispersion homogène de la méthionine dans la nourriture et de pouvoir doser la méthionine avec précision.

L'utilisation de la méthionine sous forme liquide présente des avantages par rapport à la forme cristallisée car sa manipulation et son dosage sont facilités.

L'utilisation de produits liquides permet de réaliser une dispersion homogène à des concentrations facilement contrôlables.

La méthionine étant peu soluble dans l'eau, elle ne peut pas être utilisée telle quelle en solution aqueuse du fait des volumes considérables qu'il serait nécessaire de manipuler. Toutefois, les sels de métaux alcalins de la méthionine, et en particulier le sel de sodium, dont la solubilité dans l'eau est plus importante, permettent d'obtenir des solutions suffisamment concentrées qui conviennent particulièrement bien pour la réalisation du but recherché.

Des solutions du sel de sodium de la méthionine (méthioninate de sodium) peuvent être préparées par dissolution de la méthionine dans une solution aqueuse de soude. Cependant cette façon de procéder n'est pas économiquement avantageuse du fait qu'il faut préalablement isoler la méthionine avant de la dissoudre dans un milieu approprié.

Il est également connu, d'après les demandes de brevet français FR 2 499 560, FR 2 499 563, FR 2 499 564, FR 2 499 565 et FR 2 499 566 de préparer des solutions aqueuses de méthioninate de sodium par saponification de la (β-methylmercaptoéthyl)-5 hydantoïne au moyen d'un excès de soude et/ou de carbonate de sodium et/ou de chaux. Cependant au cours de la saponification, il se forme des quantités importantes de carbonate de sodium et/ou de calcium qu'il est nécessaire d'éliminer par des traitements appropriés (concentration, précipitation, filtration,...). De plus ces méthodes conduisent à l'obtention d'un carbonate qui n'est pas directement valorisable et qui nécessite un traitement supplémentaire pour récupérer le gaz carbonique nécessaire à la synthèse de la (β-mercaptoéthyl)-5 hydantoïne.

Il a maintenant été trouvé, et c'est ce qui fait l'objet de la présente invention, que l'on peut obtenir directement une solution aqueuse de méthioninate de sodium pratiquement exempte de sels minéraux par traitement du milieu de saponification de la (β-méthylmercaptoéthyl)-5 hydantoïne dans des conditions déterminées de façon à éliminer la quasi totalité des sels minéraux sous forme de sulfate de sodium, à récupérer le gaz carbonique qui peut être recyclé et à obtenir une solution de méthioninate de sodium de concentration convenable qui peut être directement utilisée dans l'alimentation des animaux.

Après saponification de la (β-méthylmercapto-éthyl)-5 hydantoïne au moyen de soude, le milieu contient de la méthionine, essentiellement sous forme de méthioninate de sodium et du carbonate de sodium en quantité pratiquement équivalente.

Lors du traitement d'une solution de carbonate de sodium par l'acide sulfurique à une température élevée, voisine par exemple de 100°C, il se produit simultanément la neutralisation du carbonate de sodium en bicarbonate de sodium, la décomposition thermique du bicarbonate de sodium en carbonate de sodium et en gaz carbonique et la formation de sulfate de sodium.

Par ailleurs, il est connu que la solubilité du sulfate de sodium dans une solution concentrée de méthioninate de sodium varie peu avec la température.

Le procédé selon la présente invention consiste à ajouter lentement, à une température voisine de 100°C, au milieu issu de la saponification de la (β-mercaptoéthyl)-5 hydantoïne la quantité d'acide sulfurique nécessaire à la neutralisation complète du carbonate de sodium puis à concentrer le milieu de façon à avoir une teneur suffisante en méthioninate de sodium tout en récupérant le gaz carbonique et à séparer de la solution de méthioninate de sodium le sulfate de sodium qui précipite par filtration.

Le procédé selon la présente invention peut aussi être mis en oeuvre en neutralisant au moyen d'acide sulfurique tout le sodium présent dans le milieu issu de la saponification de la (β-méthylmercapto-éthyl)-5 hydantoïne (méthioninate de sodium et carbonate de sodium) puis en salifiant la méthionine par la quantité suffisante de soude et, après concentration et récupération du gaz carbonique, en séparant le sulfate de sodium précipité de la solution de méthioninate de sodium.

La solubilité du sulfate de sodium dans une solution concentrée de méthioninate de sodium variant peu avec la température, la séparation du sulfate de sodium par filtration peut être effectuée avantageusement à température élevée (voisine de 100°C). De ce fait, la filtration est facilitée étant donné que la viscosité du milieu est plus faible qu'à température plus basse.

La solution de méthioninate de sodium obtenue selon le procédé de la présente invention contient une proportion de sulfate de sodium qui est faible et, généralement inférieure à 1 % (p/v). Par ailleurs, le procédé selon l'invention permet d'obtenir, comme sous-produit, du sulfate de sodium practiquement pur qui est directement valorisable sans purification ultérieure.

Les exemples suivants, donnés à titre non limitatif, montrent comment l'invention peut être mise en pratique.

### EXEMPLE 1

A 1000 g de milieu de saponification de la (β-méthylmercaptoéthyl)-5 hydantoïne contenant 164,6 g de méthionine (soit 1,105 mole) sous forme de sel de sodium et 98,32 g de carbonate de sodium, on ajoute, à une température voisine de 100°C, une quantité d'acide sulfurique suffisante pour neutraliser le carbonate de sodium à une vitesse telle que le pH du milieu (mesuré à une température voisine de 20°C) ne soit pas inférieur à 10. Le gaz carbonique qui se dégage est récupéré. La solution obtenue est concentrée de façon que la teneur en méthioninate de sodium soit comprise entre 46 et 55 %. Le sulfate de sodium qui précipite est séparé par filtration à 100°C. On obtient ainsi un filtrat dont le poids est de 325 g et dont la composition est la suivante :
- méthioninate de sodium : 52 % (p/v)
- sulfate de sodium : 0,52 % (p/v)
Le taux de récupération du gaz carbonique est de 96%.

EXEMPLE 2

A 1000 g de milieu de saponification de la (β-méthylmercaptoéthyl)-5 hydantoïne contenant 164,3 g de méthionine (soit 1,103 mole) sous forme de sel de sodium et au total 2,84 atome-gramme de sodium, on ajoute lentement, à une température voisine de 100°C, une quantité d'acide sulfurique suffisante pour neutraliser la totalité du sodium. On récupère le gaz carbonique. On chauffe à 100°C pendant encore 1 heure afin d'éliminer le gaz carbonique résiduel. On ajoute alors 62,5 cm3 de soude à 50% (p/v), ce qui représente la quantité suffisante pour neutraliser la méthionine. La solution est concentrée de façon que la teneur en méthioninate de sodium soit comprise entre 46 et 52 %. Le sulfate de sodium qui précipite est séparé à chaud par filtration. On obtient ainsi un filtrat dont le poids est de 342 g et la composition est la suivante :
- méthioninate de sodium : 51,2% (p/v)
- sulfate de sodium : 0,61% (p/v)
Le taux de récupération du gaz carbonique est de 99%.

**Revendication**

Procédé de préparation d'une solution aqueuse concentrée du sel de sodium de la méthionine pratiquement exempte de sels minéraux à partir d'un milieu de saponification de la (β-méthylmercaptoéthyl)-5 hydantoïne par la soude contenant le sel de sodium de la méthionine et une quantité pratiquement équivalente de carbonate de sodium caractérisé en ce que l'on ajoute une quantité suffisante d'acide sulfurique pour neutraliser la totalité du carbonate de sodium et, éventuellement le méthioninate de sodium, récupère le gaz carbonique formé, concentre le milieu réactionnel, après avoir éventuellement ajouté une quantité suffisante de soude pour salifier la méthionine, de façon à avoir une concentration suffisante en sel de sodium de la méthionine, sépare le sulfate de sodium précipité par filtration et obtient ainsi un filtrat contenant le sel de sodium de la méthionine pratiquement exempt de sels minéraux et directement utilisable dans l'alimentation des animaux.

**Claim**

Process for the preparation of a concentrated aqueous solution of the sodium salt of methionine, practically free from inorganic salts, from a mixture from saponification of 5-(β-methylmercaptoethyl)-hydantoin with sodium hydroxide, containing the sodium salt of methionine and a practically equivalent quantity of sodium carbonate, characterized in that a quantity of sulphuric acid sufficient to neutralize all the sodium carbonate and, where appropriate, sodium methioninate is added, the carbon dioxide gas formed is recovered, the reaction mixture is concentrated, after adding, where appropriate, a quantity of sodium hydroxide sufficient to form a salt with the methionine, so as to have a sufficient concentration of sodium salt of methionine, the sodium sulphate precipitated is separated by filtration and a filtrate containing the sodium salt of methionine which is practically free from inorganic salts and which can be used directly in animal feedstuffs is thereby obtained.

**Patentanspruch**

Verfahren zur Herstellung einer konzentrierten, wäßrigen Lösung des Natriumsalzes des Methionins, praktisch frei von Mineralsalzen, ausgehend von einem Verseifungsmilieu des 5-(β-Methylmercaptoethyl)-hydantoins mittels Natriumhydroxid, enthaltend das Natriumsalz des Methionins und eine praktisch äquivalente Menge Natriumcarbonat, dadurch gekennzeichnet, daß man eine ausreichende Menge Schwefelsäure zugibt, um die Gesamtheit des Natriumcarbonats und gegebenenfalls das Natriummethioninat zu neutralisieren, das gebildete Kohlendioxidgas gewinnt, das Reaktionsmilieu einengt, nachdem gegebenenfalls eine ausreichende Menge Natronlauge zur Salzbildung des Methionins zugesetzt wurde, so daß eine ausreichende Konzentration an Natriumsalz des Methionins vorliegt, das ausgefallene Natriumsulfat durch Filtrieren abtrennt und man so ein Filtrat erhält, das das Natriumsalz des Methionins praktisch frei von Mineralsalzen und direkt in der Fütterung für Tiere verwendbar ist, erhält.